# EUROPÄISCHE PATENTSCHRIFT

(11) **EP 0 744 401 B1**
(45) Veröffentlichungstag und Bekanntmachung des Hinweises auf die Patenterteilung: **21.11.2001**
(21) Anmeldenummer: 96108171.8
(22) Anmeldetag: 22.05.1996
(51) Int. Cl.: C07D 241/04, B01J 31/22

(54) **Verfahren zur Herstellung von optisch aktiven 2-Piperazincarbonsäurederivaten**
Process for the preparation of optically active 2-piperazine carboxylic acid derivatives
Procédé pour la préparation des dérivés de l'acide 2-pipérazinique optiquement actif

(30) Priorität: 23.05.1995 CH 152095; 19.12.1995 CH 358195
(43) Veröffentlichungstag der Anmeldung: 27.11.1996
(60) Teilanmeldung: 01106243.7
(73) Patentinhaber: LONZA AG, CH-3945 Gampel/Wallis (CH)
(72) Erfinder: Fuchs, Rudolf, Dr., 3950 Sion (Kanton Wallis) (CH); Roduit, Jean-Paul, Dr., 3979 Grône (Kanton Wallis) (CH)
(74) Vertreter: Weinhold, Peter, Dr.rer.nat. Dipl.-Chem.

(56) Entgegenhaltungen:
- EP-A- 0 564 406
- EP-A- 0 612 758
- EP-A- 0 646 590
- WO-A-95/02583
- WO-A-95/02584
- PATENT ABSTRACTS OF JAPAN vol. 13, no. 339 (C-624) [3687] , 31.Juli 1989 & JP 01 117869 A (HOKURIKU SEIYAKU CO LTD), 10.Mai 1989,
- HELVETICA CHIMICA ACTA, Bd. XLIII, Nr. 117, 1960, Seiten 888-896, XP000561944 E. FELDER ET AL.: "ÜBER DIE KATALYTISCHE HYDRIERUNG VON PYRAZINCARBONSÄUREN"

## Beschreibung

Die vorliegende Erfindung betrifft ein Verfahren zur Herstellung von neuen und bekannten optisch aktiven 2-Piperazincarbonsäurederivaten, insbesondere von Estem und Amiden der (*R*)- oder (*S*)-2-Piperazincarbonsäure, welche gegebenenfalls an den Ringstickstoffatomen Substituenten tragen und durch die allgemeine Formel 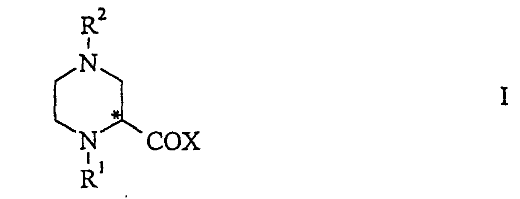 worin
R¹ und R² unabhängig voneinander jeweils Wasserstoff, C₁₋₄-Alkyl, gegebenenfalls substituiertes C₁₋₆-Alkanoyl, C₂₋₆-Perfluoralkanoyl, Aroyl, Arylalkyl, C₁₋₆-Alkoxycarbonyl, Aryloxycarbonyl, Carbamoyl oder eine Aminoschutzgruppe bedeuten und X eine Hydroxygruppe, eine C₁₋₆-Alkoxygruppe oder eine Gruppe der Formel -NR³R⁴ ist, worin wiederum
**(i)** R³ und R⁴ unabhängig voneinander Wasserstoff, C₁₋₆-Alkyl, C₃₋₆-Cycloalkyl, Aryl oder eine Aminoschutzgruppe bedeuten oder
**(ii)** R³ und R⁴ zusammen mit dem Stickstoffatom einen gegebenenfalls substituierten 5- oder 6-gliedrigen gesättigten heterocyclischen Ring bilden oder
**(iii)** R³ Wasserstoff und R⁴ eine Gruppe der Formel 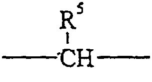 COOR⁶ ist, worin R⁶ Wasserstoff, C₁₋₄-Alkyl oder Aryl und R⁵ Wasserstoff oder die Seitengruppe einer Aminosäure bedeuten,
   repräsentiert werden, durch asymmetrische Hydrierung der entsprechenden 1,4,5,6-Tetrahydro-2-pyrazincarbonsäurederivate in Gegenwart von optisch aktiven, Rhodiumkomplexen
   Verbindungen dieser Gruppe sind wertvolle Zwischenprodukte, insbesondere für die Herstellung von pharmazeutischen Wirkstoffen. So sind beispielsweise einige bekannte Verbindungen mit X = NH-*t*-Bu und (*S*)-Konfiguration Bausteine für HIV-Protease-Inhibitoren (EP-A 541 168).

Die Erfindung betrifft weiterhin neue 1,4,5,6-Tetrahydro-2-pyrazincarbonsäurederivate als Ausgangsmaterial für die asymmetrische Hydrierung und ein Verfahren zu deren Herstellung.

Hier und im folgenden sind unter C₁₋ₙ-Alkyl jeweils geradkettige oder verzweigte primäre, sekundäre oder tertiäre Alkylgruppen mit 1 bis n Kohlenstoffatomen zu verstehen, also beispielsweise unter C₁₋₆-Alkyl Gruppen wie Methyl, Ethyl, Propyl, Butyl, Pentyl, Hexyl, Isopropyl, Isobutyl, *tert*-Butyl, Isopentyl und Neopentyl. Entsprechend sind unter C₁₋ₙ-Alkoxy und C₁₋ₙ-Alkoxycarbonyl die aus C₁₋ₙ-Alkyl und Sauerstoff bzw. Sauerstoff und Carbonyl zusammengesetzten Gruppen zu verstehen. Unter gegebenenfalls substituiertem C₁₋ₙ-Alkanoyl (C₁₋ₙ-Acyl) sind geradkettige oder verzweigte Alkanoylgruppen mit 1 bis n Kohlenstoffatomen (inclusive Carbonylkohlenstoff) zu verstehen, die noch Substituenten wie beispiels weise C₁₋₄-Alkoxygruppen tragen können, also beispielsweise unter C₁₋₆-Alkanoyl Gruppen wie Formyl, Acetyl; Methoxyacetyl, Propionyl, Butryl, Isobutyryl, Valeryl oder Caproyl. Analog sind unter C₂₋ₙ-Perfluoralkanoyl die entsprechenden perfluorierten Acylgruppen wie beispielsweise Trifluoracetyl oder Pentafluorpropionyl zu verstehen. Unter Arylalkyl sind insbesondere Gruppen wie Benzyl oder Phenethyl zu verstehen, unter Aryl insbesondere Phenyl oder substituiertes Phenyl und unter Aroyl insbesondere Benzoyl oder substituiertes Benzoyl. Unter C₃₋₆-Cycloalkyl sind Cyclopropyl, Cyclobutyl, Cyclopentyl oder Cyclohexyl zu verstehen. Unter Aminoschutzgruppen sind die in der Peptidchemie üblichen Schutzgruppen wie beispielsweise Benzyloxycarbonyl (Z) oder *tert*-Butoxycarbonyl (Boc) zu verstehen. Der Ausdruck "gesättigter heterocyclischer Ring" umfasst auch Ringe mit mehreren, gegebenenfalls auch mehreren verschiedenen Heteroatomen wie beispielsweise Morpholin. Unter dem Begriff "Seitengruppen einer Aminosäure" sind die in der allgemeinen Aminosäureformel R-CH(NH₂)-COOH mit R bezeichneten Reste zu verstehen, insbesondere diejenigen der natürlichen Aminosäuren.

Bisher bekannte Synthesen von Verbindungen der Formel I gehen von der Stammverbindung (R¹ = R² = H, X = OH) aus, die durch klassische Racematspaltung in optisch aktiver Form aus dem Racemat erhalten werden kann (E. Felder et al., *Helv*. *Chim*. *Acta* 1960, 43, 888-896). Zunächst werden die Ringstickstoffatome geschützt und dann die freie Carboxylgruppe in das Amid übergeführt. Das Verfahren ist allenfalls für den Labormassstab geeignet und erfordert der Verwendung teurer Reagenzien. WO 95/02584 A offenbart die Synthese von Verbindungen der Formel I, die durch klassische Racematspaltung von 2-tert-Butylcarbamoylpiperazinen erhalten werden. Aufgabe der vorliegenden Erfindung war daher, ein auch in technischem Massstab durchführbares Verfahren bereitzustellen, das ohne Racematspaltung auskommt.
Erfindungsgemäss wird diese Aufgabe durch das Verfahren gemäss Patentanspruch 1 gelöst.

Es wurde gefunden, dass sich die den Zielverbindungen entsprechenden 1,4,5,6-Tetrahydro-2-pyrazincarbonsäurederivate der allgemeinen Formel 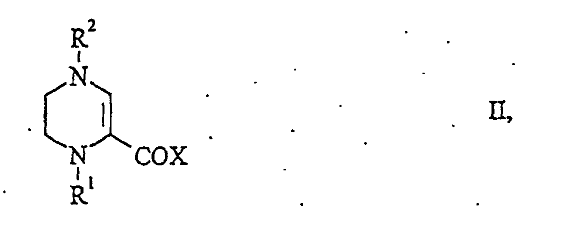 worin R¹, R² und X die oben genannten Bedeutungen haben, mit Wasserstoff in Gegenwart von katalytisch wirksamen optisch aktiven Rhodiumkomplexen
asymmetrisch zu den Zielverbindungen hydrieren lassen. Vorzugsweise werden solche 1,4,5,6-Tetrahydro-2-pyrazincarbonsäurederivate eingesetzt, in denen R¹ und/oder R² jeweils C₁₋₆-Alkanoyl, C₂₋₆-Perfluoralkanoyl, *tert*-Butoxycarbonyl ("Boc") oder Benzyloxycarbonyl ("Z") ist.
Ganz besonders bevorzugt werden solche 1,4,5,6-Tetrahydro-2-pyrazincarbonsäurederivate eingesetzt, in denen R¹ und/oder R² jeweils Formyl oder Trifluoracetyl ist Diese Derivate haben den Vorteil, dass sich die Substituenten R¹ und R² gegebenenfalls selektiv abspalten lassen, nämlich die Formylgruppe unter sauren und die Trifluoracetylgruppe unter basischen Bedingungen.
Ebenfalls vorzugsweise werden als 1,4,5,6-Tetrahydro-2-pyrazincarbonsäurederivate die Amide eingesetzt, in denen X eine Gruppe der Formel -NR³R⁴ ist und R³ und R⁴ die obenstehenden Bedeutungen haben.
Besonders bevorzugt sind solche Amide, in denen R³ Wasserstoff und R⁴ eine C₁₋₆-Alkylgruppe, insbesondere eine *tert*-Butylgruppe ist.

Als katalytisch wirksamer optisch aktiver Rhodiumkomplex wird ein Rhodiumkomplex eingesetzt, der durch Reaktion eines Rh(I)-Komplexes mit einem optisch aktiven Metallocenylphosphin gebildet wird.

Als optisch aktive Metallocenylphosphine werden vorzugsweise Verbindungen der allgemeinen Formel 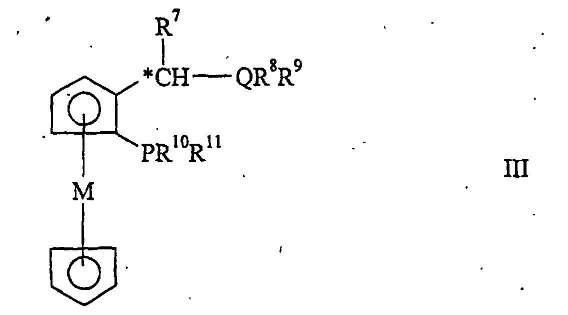 worin M Eisen(II) oder Ruthenium(II), Q Stickstoff oder Phosphor, R⁷ eine C₁₋₄-Alkylgruppe und R⁸ bis R¹¹ unabhängig voneinander C₁₋₈-Alkyl, C₅₋₈-Cycloalkyl, Phenyl oder substituiertes Phenyl bedeuten, eingesetzt.
Besonders bevorzugt sind die Metallocenylphosphine, in denen M Eisen ist, d. h. die Ferrocenylphosphine. Ebenfalls besonders bevorzugt sind die Metallocenylphosphine, in denen Q Phosphor ist, also Metallocenyldiphosphine.
Weiterhin besonders bevorzugt sind Metallocenylphosphine, in denen R⁷ Methyl ist und R⁸ und R⁹ gleich sind und *tert*-Butyl oder Cyclohexyl bedeuten. Zu diesen letztgenannten Metallocenylphosphinen zählen beispielsweise das 1-[1-(Di*-tert*-butylphosphino)ethyl]-2-(diphenylphosphino)ferrocen und das 1-[1-(Dicyclohexylphosphino)ethyl]-2-(diphenylphosphino)ferrocen. Die Herstellung dieser Verbindungen ist in EP-A 0 564 406 beschrieben. Weitere optisch aktive Metallocenylphosphine sind beispielsweise in T. Hayashi et al., *J*. *Am*. *Chem*. *Soc*. **1994,** *116*, 4221-4226, in EP-A 0 612 758 EP-A 0 546 590 und in T. Hayashi et al., *Bull*. *Chem. Soc*. *Jpn.* **1980,** *53*, 1138-1151 beschrieben.

Als Rh(I)-Komplexe, welche mit den optisch aktiven Metallocenylphosphinen zusammen die katalytisch wirksamen optisch aktiven Rhodiumkomplexe bilden, werden vorzugsweise neutrale zweikernige Komplexe der allgemeinen Formel

[Rh(L)A]₂ IV

eingesetzt. Hierbei steht L für ein C₄₋₁₂-Dien oder zwei C₂₋₁₂-Alkenmoleküle. A bedeutet Chlor, Brom oder Iod, vorzugsweise Chlor oder Brom.
Ebenfalls bevorzugt sind Rh(I)-Komplexe der allgemeinen Formel

[Rh(L)₂]⁺B⁻ V

worin L die oben genannte Bedeutung hat und B⁻ das Anion einer Sauerstoffsäure oder Komplexsäure ist Unter Anionen von Sauerstoffsäuren sind beispielsweise Anionen wie ClO₄⁻, SO₃F⁻, CH₃SO₃⁻ oder CF₃SO₃⁻ zu verstehen, unter Anionen von Komplexsäuren solche wie beispielsweise BF₄⁻, PE₆⁻, AsF₆⁻, SbF₆⁻ oder SbCl₆⁻.

Besonders bevorzugt als Liganden L in den Rh(I)-Komplexen IV und V sind Diene, insbesondere Norbornadien und 1,5-Cyclooctadien.
Diese Komplexe sind bekannt, beispielsweise aus EP-A 0 302 021 oder US-A 5 011 995.

Die asymmetrische Hydrierung der 1,4,5,6-Tetrahydropyrazincarbonsäurederivate II wird vorteilhaft bei einer Temperatur von 20 bis 200 °C und einem Wasserstoffdruck von 1 bis 200 bar durchgeführt. Das molare Verhältnis Katalysator-Edukt liegt vorteilhaft bei 1:100 bis 1:5000, vorzugsweise bei 1:1000 bis 1:2000.
Als Lösungsmittel für die asymmetrische Hydrierung eignen sich beispielsweise Wasser, niedrige Alkohole wie Methanol, aromatische Kohlenwasserstoffe wie Toluol, Ketone wie Aceton oder Carbonsäureester wie Ethylacetat.

Die 1,4,5,6-Tetrahydropyrazincarbonsäurederivate II sind neue Verbindungen und ebenfalls Gegenstand der vorliegenden Erfindung.
Sie können beispielsweise dadurch hergestellt werden, dass ein entsprechendes Pyrazincarbonsäurederivat der allgemeinen Formel 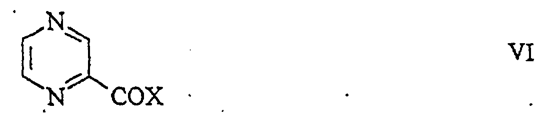 worin X die oben genannte Bedeutung hat, mit Wasserstoff an einem Palladiumkatalysator partiell zum 1,4,5,6-Tetrahydro-2-pyrazincarbonsäurederivat der allgemeinen Formel 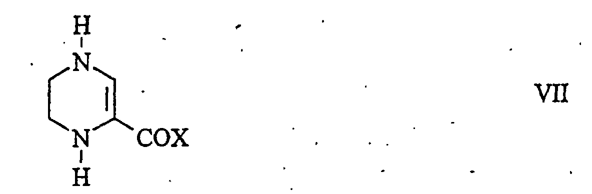 worin X die oben genannte Bedeutung hat, hydriert und anschliessend nach einer bekannten Methode, beispielsweise durch Acylierung mit einem Carbonsäureanhydrid, in die an den Ringstickstoffen N¹ und/oder N⁴ substituierte Verbindung übergeführt wird.

Verbindungen II, in denen X eine (substituierte) Aminogruppe ist, können auch ausgehend von 1,4,5,6-Tetrahydropyrazin-2-carbonitril hergestellt werden, beispielsweise durch eine Ritter-Reaktion. Die letztgenannte Verbindung ist nach einem in EP-A 0 175 364 beschriebenen Verfahren leicht zugänglich.

Die nachfolgenden Beispiele verdeutlichen die Durchführung des erfindungsgemässen Verfahrens und die Herstellung der erfindungsgemässen Verbindungen, ohne dass darin eine Einschränkung zu sehen ist.

### Beispiel 1

### Pyrazincarbonsäuremethylester

Zu 1200 ml Methanol wurden unter Argon bei 4-6 °C innerhalb von 1 h 106,6 g Thionylchlorid zugetropft. Bei 9 °C wurden 100,1 g Pyrazincarbonsäure zugegeben und das Gemisch 2 h auf 61 °C erwärmt, wobei die Säure vollständig in Lösung ging.
Nach dem Abkühlen auf Raumtemperatur wurde langsam eine Lösung von 145 g Natriumhydrogencarbonat in 1,41 Wasser zugegeben. Das Methanol wurde am Rotationsverdampfer bei 45 °C Badtemperatur und 50-120 mbar abdestilliert und der Rückstand dreimal mit Dichlormethan (400 ml, 100 ml, 100 ml) extrahiert. Durch Einengen der organischen Phase erhielt man 83,15 g Rohprodukt, das aus ca. 250 g Diisopropylether umkristallisiert wurde.
Ausbeute: 70,6 g, zuzüglich 10,28 g aus der Mutterlauge (total 72,5%)
- ¹H NMR (CDCl₃, 400 MHz): δ =: 4,08 (s, 3H);
8,75 (d, *J* = 0,5 Hz, 1H);
8,80 (d, *J* = 0,5 Hz, 1H);
9,30 (s, 1H).

### Beispiel 2

### 1,4,5,6-Tetrahydropyrazin-2-carbonsäuremethylester

In einem 500 ml-Autoklaven wurden in 100 ml Methylacetat 7,8 g Pyrazincarbonsäuremethylester (hergestellt nach Beispiel 1) und 1,5 g Palladium auf Aktivkohle (10% Pd) vorgelegt. Der Autoklav wurde zweimal mit Stickstoff und zweimal mit Wasserstoff gespült, indem das Gas bis 8 bar Druck aufgepresst und wieder entspannt wurde. Anschliessend wurde bei 20 °C und 10 bar Wasserstoffdruck unter Rühren 9 h hydriert. Danach wurde mit Stickstoff gespült, das Reaktionsgemisch über eine Filternutsche (0,45 µm Porenweite) filtriert und der Katalysator mit Methylacetat gewaschen. Durch Einengen wurde roher 1,4,5,6-Tetrahydro-2-pyrazincarbonsäuremethylester erhalten.
- ¹H NMR (CDCl₃, 400 MHz): δ =: 3,17-3,25 (m, 2H);
3,33-3,42 (m, 2H);
3,71 (s, 3H);
6,93 (br. s, 1H).

Das Produkt wurde ohne weitere Reinigung acyliert.

### Beispiel 3

### I-Acetyl-1,4,5,6-tetrahydro-2-pyrazincarbonsäuremethylester

Zu 25,00 g (175,9 mmol) 1,4,5,6-Tetrahydro-2-pyrazincarbonsäuremethylester, 250 ml Tetrahydrofuran und 21,30 g (210,5 mmol) Triethylamin wurden bei 0 °C 15,10 g (192,5 mmol) Acetylchlorid innerhalb von 25 min zugetropft. Anschließend wurde auf Raumtemperatur erwärmt und nach 1 h 200 ml Wasser und 200 ml Essigsäureethylester zugegeben. Die Wasserphase wurde noch 3-4 mal mit insgesamt 500 ml Essigsäureethylester gewaschen und die vereinigten organischen Extrakte über Magnesiumsulfat getrocknet Nach dem Abziehen des Lösungsmittels wurden 22,30 g (69%) der Titelverbindung als gelbes Öl erhalten.
Umkristallisation aus Essigsäureethylester lieferte 1-Acetyl-1,4,5,6-tetrahydro-2-pyrazincarbonsäuremethylester als hellgelben, kristallinen Feststoff.
Schmp.: 136-139°C
- ¹H NMR (CDCl₃, 400 MHz): δ =: 2,10 (s, 3H);
3,35 (br. s, 2H);
3,00-5,00 (br. m, 2H);
3,75 (s, 3H);
5,02 (br. s, 1H);
7,32 (d, 1H).

### Beispiel 4

### 1,4-Diacetyl-1,4,5,6-tetrahydro-2-pyrazincarbonsäuremethylester

In 200 g Essigsäureanhydrid wurden 10,1 g 1,4,5,6-Tetrahydro-2-pyrazincarbonsäuremethylester 2 h auf 135 °C erhitzt. Anschliessend wurde das überschüssige Essigsäureanhydrid bei 57 °C/55 mbar abdestilliert. Der Rückstand wurde mit 100 g Dichlormethan und 20 g Wasser versetzt. Die organische Phase wurde abgetrennt, mit 30 ml Wasser gewaschen und zur

Trockne eingeengt. Als öliger Rückstand wurden 18,1 g Rohprodukt erhalten. Dieses wurde durch Mitteldruck-Säulenchromatographie (Säule 5×40 cm, Silicagel) mit Ethylacetat/Dichiormethan/Methanol (10:5:1) gereinigt.
- Ausbeute:: 11,6 g (85%)
- ¹HNMR (DMSO-d₆, 80 °C, 400 MHz): δ =: 1,98 (s, 3H);
2,25 (s, 3H);
3,60-3,70 (m, 1H);
3,70 (s, 3H);
7,60 (br. d, 1H).

### Beispiel 5

### 1-Acetyl-1,4,5,6-tetrahydro-2-pyrazincarbonsäure-tert-butylamid

Zu 100 ml (1,75 mol) Essigsäure wurden bei Raumtemperatur langsam 70 g (0,77 mol) Methansulfonsäure und anschliessend 20,0 g (184 mmol) 1,4,5,6-Tetrahydropyrazin-2-carbonitril gegeben. In diese Mischung wurde bei 25 °C 23,0 g (410 mmol) Isobuten eingeleitet und das Gemisch 5 h gerührt.
Anschliessend wurde mit 30%iger Natronlauge neutralisiert, wobei die Temperatur stets unter 30 °C gehalten wurde. Das auf pH 8-10 eingestellte Gemisch wurde dreimal mit je 200 ml Methylethylketon extrahiert. Die vereinigten organischen Phasen wurden über Magnesiumsulfat getrocknet und das Lösungsmittel abdestilliert. Der Rückstand (41,2 g) wurde in 80 ml Ethylacetat in der Hitze gelöst. Nach Abkühlung auf 20 °C wurden 500 ml Hexan zugegeben, weiter auf 0 °C abgekühlt, das ausgefallene Produkt nach 1 h abfiltriert und getrocknet.
Ausbeute: 32,6 g (79%) hellbeiges Pulver
Schmp.: 151,3-152,6 °C
- ¹H NMR (CDCl₃, 400 MHz): δ =: 2,08 (s, 3H);
3,35 (br. s, 2H);
3,74 (s, 3H);
2,00-5,00 (br. m, 2H);
7,31 (d, *J* = 7,0 Hz, 1H).
- ¹³C NMR (CDCl₃, 100 MHz): δ =: 22,38 (CH₃);
37,28 (CH₂);
42,79 (CH₂);
51,15 (OCH₃);
103,63 (C=);
13 5,94 (CH=);
165,21 (COO);
171,85 (CON).

### Beispiel 6

### 1-Propionyl-1,4,5,6-tetrahydropyrazin-2-carbonsäure-tert-butylamid

Die Verbindung wurde analog zu Beispiel 5 unter Verwendung von Propionsäure anstelle von Essigsäure hergestellt.
Ausbeute: 75%
Schmp: 172,4-172,6 °C
- ¹H NMR (CDCl₃, 400 MHz): δ =: 1,09 (t, *J* = 7,3 Hz, 3H);
1;37 (s, 9H);
2,39 (q, *J* = 7,3 Hz, 2H);
3,28 (m, 2H);
3,57 (m, 2H);
5,41 (br. s, 1H);
5,49 (br. s, 1H);
7,09 (d, *J*= 6,3 Hz, 1H).
- ¹³C NMR (CDCl₃, 100 MHz): δ =: 9,44 (CH₃);
27,55 (CH₂);
29,12 (CH₃);
38,29 (CH₂);
42,19 (CH₂);
50,92 (C);
106,66 (C);
132,20 (CH);
165,21 (C=O);
177,20 (C=O).

### Beispiel 7

### 1-Isobutyryl-1,4,5,6-tetrahydropyrazin-2-carbonsäure-tert-butylamid

Die Verbindung wurde analog zu Beispiel 5 unter Verwendung von Isobuttersäure anstelle von Essigsäure hergestellt.
Ausbeute : 48%
Schmp: 180,0-184,4 °C
- ¹H NMR (CDCl₃, 400 MHz): δ =: 1,07 (d, *J* = 6,5 Hz, 6H);
1,37 (s, 9H);
2,82 (sept, *J* = 6,5 Hz, 1H);
3,27 (m, 2H);
3,6 (br. m, 2H);
4,94 (br. s, 1H);
5,48 (br. s, 1H);
7,12 (d, *J*= 6,3 Hz, 1H).
- ¹³C NMR (CDCl₃, 100 MHz): δ =: 19,36 (CH₃);
29,07 (CH₃);
32,50 (CH);
38,39 (CH₂);
42,42 (CH₂);
50,94 (C);
106,73 (C);
132,07 (CH);
165,21 (C=O);
180,85 (C=O).

### Beispiel 8

### 1,4-Diacetyl-1,4,5,6-tetrahydro-2-pyrazincarbonsäure-tert-butylamid

Zu 20,00 g (88,8 mmol) 1-Acetyl-1,4,5,6-tetrahydro-2-pyrazincarbonsäure-*tert*-butylamid wurden 16,7 g (163 mmol) Essigsäureanhydrid und 12,4 g (157 mmol) Pyridin gegeben und das Gemisch 4 h bei 25 °C gerührt. Anschliessend wurden 100 ml Wasser zugegeben und mit 30 ml Natronlauge (20%) auf pH 6 eingestellt. Dann wurde dreimal mit je 100 ml Methylethylketon ausgeschüttelt. Die vereinigten organischen Extrakte wurden über Magnesiumsulfat getrocknet, mit Toluol versetzt und eingeengt. Das stark schäumende Rohprodukt kristallisierte und 11,40 g (48%) der Titelverbindung wurden als leicht brauner Feststoff isoliert.
Schmp.: 138,0-139,2 °C
- ¹H NMR (CDCl₃, 400 MHz): δ =: 1,41 (s, 9H);
2,14 (s, 3H);
2,32 (s, 3H);
3,71-3,80 (m, 4H);
5,82 (br. s, 1H);
7,34 (s, 1H).
- ¹³C NMR (CDCl₃, 100 MHz): δ =: 21,30 (CH₃);
21,58 (CH₃);
28,76'(CH₃);
39,44 (CH₂);
42,43 (CH₂);
51,76 (C);
116,61 (C);
123,36 (CH);
163,04 (C=O);
168,56 (C=O);
171,68 (C=O).

### Beispiel 9

### 4-Acetyl-1-propionyl-1,4,5,6-tetrahydro-2-pyrazincarbonsäure-tert-butylamid

168,7 g (0,705 mol) 1-Propionyl-1,4,5,6-tetrahydro-2-pyrazincarbonsäure-*tert*-butylamid wurden in 1,92 l Butylacetat/Acetonitril-Gemisch (4:1) vorgelegt. Die Suspension wurde auf 50 °C geheizt und 68,2 g (0,812 mol) Natriumhydrogencarbonat wurden zugegeben. Eine Lösung von 57,0 g (0,726 mol) Acetylchlorid in 120 ml Butylacetat wurde innerhalb von 30 min zugetropft. Anschliessend wurde das Reaktionsgemisch noch 40 min bei 50 °C weitergerührt. Vorsichtig wurden 190 ml Wasser zugegeben, die wässerige Phase abgetrennt und nochmals bei 50 °C mit 200 ml Butylacetat extrahiert. Die vereinigten organischen Extrakte wurden auf 1100 ml aufkonzentriert und zur Kristallisation langsam auf 2 °C gekühlt. Der Feststoff wurde abfiltriert und mit 100 ml kaltem Butylacetat nachgewaschen.

Umkristallisation aus 700 ml Butylacetat ergab 183,8 g (92,7%) der Titelverbindung als weisse Kristalle.
Schmp.: 133,5-134,6 °C
- ¹H NMR (CDCl₃, 400 MHz): δ =: 1,13 (t, *J* = 7,3 Hz, 3H);
1,40 (s, 9H);
2,32 (s, 3H);
2,38 (g, *J* = 7,3 Hz; 2H);
3,70 (m, 4H);
5,77 (br. s, 1H);
7,34 (s, 1H).
- ¹³C NMR (CDCl₃, 100 MHz): δ =: 9,27(CH₃);
21,46(CH₃);
27,88 (CH₂);
28,75 (CH₃);
39,88(CH₂);
42,55 (CH₂);
51,72 (C);
116,49(C);
123,43 (CH);
163,20 (C=O);
168,61 (C=O);
175,6 (C=O).

### Beispiel 10

### 1-Acetyl-4-benzoyl-1,4,5,6-tetrahydro-2-pyrazincarbonsäure-tert-butylamid

Analog zu Beispiel 9 wurde aus 1-Acetyl-1,4,5,6-tetrahydro-2-pyrazincarbonsäure-*tert*-butylamid und Benzoylchlorid die Titelverbindung als weisser kristalliner Feststoff erhalten.
Schmp.: 157,0-157,5 °C
- ¹H NMR (CDCl₃, 400 MHz): δ =: 1,37 (s; 9H);
2,17 (s, 3H);
3,78 (m, 2H);
3,89 (m, 2H);
5,74 (br. s, 1H);
7,50 (m, 6H).
- ¹³C NMR (CDCl₃, 100 MHz): δ =: 22,63 (CH₃);
28,74 (CH₃);
39,41 (CH₂);
44,05 (CH₂);
51,70 (C);
116,98 (C);
124,19 (C);
128,20 (CH);
128,87 (CH);
131,43 (CH);
133,33 (CH);
163,00 (C=O);
169,48 (C=O);
171,51 (C=O).

### Beispiel 11

### 1-Acetyl-4-methoxycarbonyl-1,4,5,6-tetrahydro-2-pyrazincarbonsäure-tert-butylamid

Analog zu Beispiel 9 wurde aus 1-Acetyl-1,4,5,6-tetrahydro-2-pyrazincarbonsäure-*tert*-butylamid und Chlorameisensäuremethylester die Titelverbindung als weisser kristalliner Feststoff erhalten.
Schmp.: 134,0-135,3 °C
- ¹H NMR (CDCl₃, 400 MHz): δ =: 1,40 (s, 9H);
2,12 (s, 3 H);
3,67 (m, 4H);
3,84 (s, 3H);
5,70 (br. s, 1H);
7,50 (br. s, 1H).
- ¹³C NMR (CDCl₃, 100 MHz): δ =: 22,54 (CH₃);
28,77 (CH₃);
39,07 (CH₂);
44,05 (CH₂);
51,63 (C);
53,86 (CH₃);
116,32 (C);
122,48 (CH);
152,93 (C=O);
163,26 (C=O);
171,72 (C=O).

### Beispiel 12

### 1-Acetyl-4-phenoxycarbonyl-1,4,5,6-tetrahydro-2-pyrazincarbonsäure-tert-butylamid

Analog dem Beispiel 9 wurde aus 1-Acetyl-1,4,5,6-tetrahydro-2-pyrazincarbonsäure-*tert*-butylamid und Chlorameisensäurephenylester die Titelverbindung als weisser kristalliner Feststoff erhalten.
Schmp.: 156,4-157,5 °C
- ¹H NMR (CDCl₃, 400 MHz): δ =: 1,41 (s, 9H);
2,16 (s, 3H);
3,79 (m, 4H);
5,76 (br. s, 1H);
7,13-7,41 (m, 6H).
- ¹³C NMR (CDCl₃, 100 MHz): δ =: 22,58 (CH₃);
28,74 (CH₃);
39,20 (CH₂);
44,27 (CH₂);
51,72 (C);
117,40 (C);
121,35 (CH);
126,23 (CH);
129,59 (CH);
150,59 (C);
150,9 (C=O);
163,10 (C=O);
171,60 (C=O).

### Beispiel 13

### 1,4-Dipropionyl-1,4,5,6-tetrahydro-2-pyrazincarbonsäure-tert-butylamid

Analog zu Beispiel 9 wurde aus 1-Propionyl-1,4,5,6-tetrahydro-2-pyrazincarbonsäure*-tert*-butylamid und Propionylchlorid die Titelverbindung als weisser kristalliner Feststoff erhalten.
Schmp.: 131,0-132,8 °C
- ¹H NMR (CDCl₃, 400 MHz): δ =: 1,13 (t, *J* = 7,3 Hz, 3H);
1,20(t, *J* =7,3 Hz, 3H);
1,40 (s, 9H);
2,38 (q, *J*= 7,3 Hz, 2H);
2,60 (q, *J* = 7,3 Hz, 2H);
3,70 (m, 4H);
5,77 (br. s, 1H);
7,39 (s, 1H).
- ¹³C NMR (CDCl₃, 100 MHz): δ =: 8,81 (CH₃);
9,27 (CH₃);
26,68 (CH₂);
27,85 (CH₂);
28,76 (CH₃);
39,88 (CH₂);
42,67 (CH₂);
51,69 (C);
116,29 (C);
122,86 (CH);
163,29 (C=O);
171,93 (C=O);
175,49 (C=O).

### Beispiel 14

### 4-Acetyl-1-isobutyryl-1,4,5,6-tetrahydro-2-pyrazincarbonsäure-tert-butylamid

Analog zu Beispiel 9 wurde aus 1-Isobutyryl-1,4,5,6-tetrahydro-2-pyrazincarbonsäure*-tert*-butylamid und Acetylchlorid die Titelverbindung als weisser kristalliner Feststoff erhalten.
Schmp.: 116,3-117,2°C
- ¹H NMR (CDCl₃, 400 MHz): δ =: 1,10 (d, *J*= 6,6 Hz; 6H);
1,40 (s, 9H);
2,33 (s, 3H);
2,72 (m, 1H);
3,70 (m, 4H);
5,80 (br. s, 1H);
7,37 (s, 1H).
- ¹³C NMR (CDCl₃, 100 MHz): δ =: 19,33 (CH₃);
21,46 (CH₃);
28,67 (CH₃);
33,04 (CH);
39,81 (CH₂);
42,67 (CH₂);
51,65 (C);
116,17 (C);
123,49 (CH);
163,32 (C=O);
168,60 (C=O);
179,11 (C=O).

### Beispiel 15

### 1-Acetyl-4-methoxyacetyl-1,4,5,6-tetrahydro-2-pyrazincarbonsäure-tert-butylamid

Analog zu Beispiel 9 wurde aus 1-Acetyl-1,4,5,6-tetrahydro-2-pyrazincarbonsäure-*tert*-butylamid und Methoxyacetylchlorid die Titelverbindung als weisser kristalliner Feststoff erhalten.
Schmp.: 109,6-110,7 °C
- ¹H NMR (CDCl₃, 400 MHz): δ =: 1,41 (s, 9H);
2,14 (s, 3H);
3,46 (s, 3H);
3,73 (m, 4H);
4,32 (s, 2H);
5,73 (br. s, 1H);
7,32 (s, 1H).
- ¹³C NMR (CDCl₃, 100 MHz): δ =: 22,60 (CH₃);
28,76 (CH₃);
39,33 (CH₂);
42,67 (CH₂);
51,82 (C);
59,51 (CH₃);
71,31 (CH₂);
117,34 (C);
121,88 (CH);
162,89 (C=O);
167,26 (C=O);
171,62 (C=O);

### Beispiel 16

### 1-Propionyl-4-trifluoracetyl-1,4,5,6-tetrahydro-2-pyrazincarbonsäure-tert-butylamid

Analog zu Beispiel 9 wurde aus 1-Propionyl-1,4,5,6-tetrahydro-2-pyrazincarbon-säure*-tert*-butylamid und Trifluoracetylchlorid die Titelverbindung als weisser kristalliner Feststoff erhalten.
Schmp.: 132,6-133,3 °C
- ¹HNMR(C₂D₂Cl₄, 400 MHz, 100 °C): δ =: 1,13 (t, *J*= 7, Hz, 3H);
1,38 (s, 9H);
2,35 (q, J= 7,3 Hz, 2H);
3,80 (m. 4H);
5,66 (br. s, 1H);
7,28 (br. s, 1H).
- ¹³CNMR (C₂D₂Cl₄, 100 MHz, 100 °C): δ =: 9,15 (CH₃);
27,86 (CH₂);
28,71 (CH₃);
40,10 (CH₂);
44,90 (CH₂);
51,88 (C);
115,87 (q, ¹*J*_{CF} = 286 Hz, CF₃);
117,75 (CH);
122,05 (C);
154,47 (q, ²*J*_{CF} = 38,7 Hz, COCF₃);
162,17 (CONH);
173,96 (COEt).

### Beispiel 17

### 4-Formyl-1-propionyl-1,4,5,6-tetrahydro-2-pyrazincarbonsäure-tert-butylamid

### Verfahren A: Formylierung mit in situ generiertem Formylchlorid

Zu einer Suspension von 70,00 g (0,293 mol) l-Propionyl-1,4,5,6-tetrahydro-2-pyrazincarbonsäure-*tert*-butylamid in 350 ml Butylacetat wurde bei 20 °C 70,00 g (1,521 mol) Ameisensäure zugegeben. Zu der erhaltenen gelblichen Lösung wurde innerhalb von 4,5 h eine Lösung aus 63 g Thionylchlorid in 140 ml Butylacetat zugetropft. Die Temperatur wurde dabei unter 25 °C gehalten. Die Reaktionslösung wurde mit 50 ml 5%iger Natriumhydrögencarbonatlösung gewaschen und die wässerige Phase mit 100 ml Dichlormethan nachgewaschen. Beide organische Phasen wurden zusammengegeben und nach dem Entfernen des Lösungsmittels der Rückstand aus Butylacetat umkristallisiert. 71,2 g (82,7%) der Titelverbindung wurden als weisser kristalliner Feststoff erhalten.

### Verfahren B: Formylierung mit Acetyl-formyl-anhydrid (analog Lit.: J.C. Sheehan, D.-D. Yang, J. Am. Chem. Soc. 1958, 80, 1154.)

Zu einer Lösung von 31,2 g (0,13 mol) 1-Propionyl-1,4,5,6-tetrahydro-2-pyrazincarbonsäure-*tert*-butylamid in 40,0 g (0,87 mol) Ameisensäure wurde bei 30 °C ein Gemisch aus 20,0 g (0,196 mol) Essigsäureanhydrid und 18,00 g (0,391 mol) Ameisensäure innerhalb von 30 min zugetropft. Das Reaktionsgemisch wurde noch 20 min bei 30 °C gerührt. Dann wurden 50 ml Wasser zugegeben und die klare Lösung 50 min bei 40 °C gehalten. Das Produkt wurde extrahiert und aus *n*-Butylacetat kristallisiert 30,75 g (88%) der Titelverbindung wurden als weisser kristalliner Feststoff erhalten.
Schmp.: 132,1-133,5 °C
- ¹H NMR (CDCl₃, 400 MHz): δ =: 1,14 (t, *J*= 7,3 Hz, 3H);
1,40 (s, 9H);
2,39 (q, *J* = 7,3 Hz, 2H);
3,66 (m, 2H);
3,74 (m, 2H);
5,75 (br. s, 1H);
7,22 (s, 1H);
8,41 (s, 1H).
- ¹³C NMR (CDCl₃, 100 MHz): δ =: 9,25 (CH₃);
27,89 (CH₂);
28,67 (CH₃);
39,25 (CH₂);
45,06 (CH₂);
51,79 (C);
118,3 (C);
122,9 (CH);
161,01 (C=O);
162,79 (C=O);
175,4 (C=O).

### Beispiel 18

### 1-Acetyl-4-formyl-1,4,5,6-tetrahydro-2-pyrazincarbonsäure-tert-butylamid

Die Verbindung wurde analog zu Bsp. 17 (Verf. B) aus 1-Acetyl-1,4,5,6-tetrahydro-2-pyrazincarbonsäure-*tert*-butylamid und Ac₂O/HCOOH als weisser kristalliner Feststoff erhalten.
Schmp.: 131,0-132,9 °C
- ¹H NMR (CDCl₃, 400 MHz): δ =: 1,41 (s, 9H); 2,15 (s, 3H); 3,70 (m, 4H); 5,75 (br. s, 1H); 7,21 (s, 1H); 8,41 (s, 1H).
- ¹³C NMR (CDCl₃, 100 MHz): δ =: 22,62 (CH₃); 28,73 (CH₃); 38,65 (CH₂); 44,99 (CH₂); 51,85 (C); 118,27 (C); 122,90 (CH); 160,94 (C=O); 162,63 (C=O); 171,7 (C=O).

### Beispiel 19

### (S)-1,4-Diacetyl-2-piperazincarbonsäure-tert-butylamid

Ein Autoklav wurde unter Sauerstoffausschluss mit 10 g (37 mmol) 1,4-Diacetyl-1,4,5,6-tetrahydro-2-pyrazincarbonsäure-*tert*-butylamid, 20,3 mg (37 µmol) 1-[1(*R*)-(Di-*tert*-butylphosphino)ethyl]-2(*S*)-(diphenylphosphino)ferrocenund 9,2 mg (20 µmol) Bicyclo[2.2.1]hepta-2,5-dienrhodium(I)chlorid-Dimer versehen. Nach Spülen mit Argon wurden 80 ml sauerstofffreies Methanol zugegeben. Bei einem Anfangsdruck von 50 bar und einer Temperatur von 110°C wurde 20 h hydriert. Der Autoklav wurde entspannt und mit Stickstoff gespült. Das Lösungsmittel des Reaktionsgemisches wurde vollständig abdestilliert und man erhielt so 10,50 g (95%) des Titelproduktes. Die GC-Analyse zeigte einen Umsatz von 85%. Die Enantioselektivität der Hydrierung wurde nach Abspaltung der Schutzgruppen mit Salzsäure durch GC-Analyse zu 90,5% ee bestimmt.

### Beispiel 20

### (S)-1,4-Diacetyl-2-piperazincarbonsäure-tert-butylamid

Ein Autoklav wurde unter Sauerstoffausschluss mit 8,00 g (29,9 mmol) 1,4-Diacetyl-1,4,5,6-tetrahydro-2-pyrazincarbonsäure-*tert*-butylamid, 10,0 mg (18 µmol) 1-[1(*R*)-(Di-*tert*-butylphosphino)ethyl]-2(*S*)-(diphenylphosphino)ferrocen und 6,0 mg (16 µmol) Bis(bicyclo[2.2.1]-hepta-2,5-dien)-rhodium(I)-tetrafluoroboratversehen. Nach Spülen mit Argon wurden 80 ml sauerstofffreies Methanol zugegeben. Bei einem Anfangsdruck von 50 bar und einer Temperatur von 110°C wurde 20 h hydriert. Nach Aufarbeitung des Reaktionsgemisches analog zu Beispiel 19 erhielt man 10,50 g (75%) des Titelproduktes. Die GC-Analyse zeigte einen Umsatz von 97%. Die Enantioselektivität der Hydrierung wurde nach Abspaltung der Schutzgruppen mit Salzsäure durch GC-Analyse zu 90,7% ee bestimmt
Schmp.: 139,9-150,6°C
- ¹H NMR (CDCl₃, 400 MHz): δ =: 1,31; 1,33 (2 s, 9H),
2,10;·2,12; 2,15; 2,17; 2,19; 2,22; 2,26 (7 s, 6H);
2,77-2,83 (m, 1H);
3,18-3,28 (m, 2H);
3,70-3,73 (m, 1H);
4,41-4,49 (m, 2H);
5,04 (br. s, 1H);
5,95 (br. s, 1H).

### Beispiel 21

### (S)-4-Formyl-1-propionyl-2-piperazincarbonsäure-tert-butylamid

Ein Autoklav wurde unter Sauerstoffausschluss mit 16,1 g (60,2 mmol) 4-Formyl-1-propionyl-1,4,5,6-tetrahydro-2-pyrazicarbonsäure-*tert*-butylamid, 19,5 mg (35,9 µmol) 1-[1(*R*)-(Di-*tert*-butylphosphino)ethyl]-2(*S*)-(diphenylphosphino)ferrocen und 11,1 mg (29,6 µmol) Bis(bicyclo[2.2.1]hepta-2,5-dien)-rhodium(I)-tetrafluoroboratversehen. Nach Spülen mit Argon wurde 80 ml sauerstofffreies Methanol zugegeben. Bei einem Anfangsdruck von 12 bar und einer Temperatur von 90°C wurde 8 h hydriert Der Autoklav wurde entspannt und mit Stickstoff gespült Das Lösungsmittel des Reaktionsgemisches wurde vollständig abdestilliert und man erhielt 17,00 g (94%) des Titelproduktes. Die GC-Analyse zeigte einen Umsatz von 99%. Die Enantioselektivität der Hydrierung wurde nach Abspaltung der Schutzgruppen mit Salzsäure durch GC-Analyse zu 96,5% ee bestimmt.
Schmp.: 132,1-134,3 °C
- ¹H NMR (CDCl₃, 400 MHz): δ =: 1,21 (t, *J*= 7,3 Hz, 3H);
1,30; 1,31 (2s, 9H);
2,46 (q, *J* = 7,3 Hz, 2H);
2,75-2,82 (m, 1H);
3,12-3,1.8 (m, 2H);
3,60-3,78 (m, 1H);
4,21-4,38 (m, 2H);
5,07 (br. s, 1H);
5,92 (br. s, 1H);
8,10; 8,18 (2 s, 1H).

### Beispiele 22-41

Analog zu Beispiel 19 wurden verschiedene Tetrahydropyrazin-Derivate II in Anwesenheit von 1-[1(*R*)-(Di-*tert*-butylphosphino)ethyl]-2(*S*)-(diphenylphosphino)ferrocen und [Rh(cod)Cl]₂ (A) oder Rh(nbd)₂BF₄ (B) in Methanol hydriert (cod = 1,5-Cyclooctadien, nbd = Bicyclo[2.2.1]heptadien). Die Ergebnisse sind in der folgenden Tabelle 1 zusammengestellt. Angegeben ist jeweils das molare Verhältnis Edukt: Rhodium, die Substituenten X, R¹ und R², die Reaktionsbedingungen, der verwendete Katalysator, der Umsatz und der Enantiomerenüberschuss (ee). Als Produkt wurde jeweils bevorzugt das (*S*)-Enantiomer gebildet.

**Tabelle 1**

| Edukt:Rh | X/R¹/R² | Temp [°C]/Zeit [h] | Druck [bar] | Katalysator | Umsatz [%] | ee [%] |
|---|---|---|---|---|---|---|
| 500 | NH-*t*-Bu/Ac/Ac | 90/4 | 50 | B | 94 | 93,7 |
| 1000 | NH-*t*-Bu/EtCO/EtCO | 90/22 | 50 | B | 91 | 87,5 |
| 1000 | NH-*t*-Bu/EtCO/Ac | 90/22 | 10 | B | 94 | 91 |
| 1000 | NH-*t*-Bu/Ac/COPh | 90/21 | 50 | B | 83 | 89,9 |
| 475 | NH-*t*-Bu/Me₂CHCO/Ac | 90/21 | 50 | B | 96,9 | 86,3 |
| 1000 | NH-*t*-Bu/Ac/COOMe | 90/20 | 50 | B | 97 | 88,3 |
| 480 | NH-*t*-Bu/Ac/CHO | 90/1 | 50 | B | 99,8 | 96,9 |
| 487 | NH-*t*-Bu/EtCO/CHO | 90/2 | 50 | B | 99,7 | 96,5 |
| 482 | NH-*t*-Bu/EtCO/CHO | 70/6 | 12 | B | 99,8 | 96,8 |
| 475 | NH-*t*-Bu/CHO/CHO | 70/6 | 12 | B | 72,4 | 91,7 |
| 400 | NH-*t*-Bu/EtCO/COCF₃ | 90/17 | 50 | B | 95 | 88,2 |
| 400 | NH-*t*-Bu/Ac/MeOCH₂CO | 70/6 | 12 | B | 99 | 84,5 |
| 250 | NH-*t*-Bu/Ac/H | 70/20 | 50 | A | 87 | 58 |
| 500 | NH-*t*-Bu/EtCO/H | 90/24 | 50 | A | 78 | 52 |
| 500 | NH-*t*-Bu/COCHMe₂/H | 90/23 | 50 | B | 74 | 41 |
| 500 | NH-*t*-Bu/COCH₂OMe/H | 90/23 | 50 | B | 39 | 56 |
| 25 | OMe/H/H | 50/20 | 50 | A | 87 | 40 |
| 250 | OMe/Ac/H | 70/20 | 50 | A | 87 | 58 |
| 500 | OMe/Ac/H | 70/22 | 50 | A | 59 | 37,8 |
| 245 | OMe/Ac/Ac | 50/20 | 50 | A | 74 | 95 |

### Beispiel 42

### 1-Trifluoracetyl-1,4,5,6-tetrahydropyrazin-2-carbonsäure-tert-butylamid

In einem 500 ml Kolben wurden 50 ml Trifluoressigsäure unter Argon vorgelegt. Bei 21 °C wurden 12,5 g Methansulfonsäure zugetropft und dann portionsweise 20 g 2-Cyan-1,4,5,6-tetrahydropyrazin-Methansulfonsäuresalz (97 mmol) zugegeben, wobei eine leichte Exothermie beobachtet wurde. Anschliessend wurden innerhalb 1 h bei 20 °C 10 g (178 mmol) Isobuten eingeleitet. Das Reaktionsgemisch wurde noch 2 h bei 20 °C gerührt, dann bei dieser Temperatur tropfenweise mit 13,3 g (111 mmol) Thionylchlorid versetzt und noch weitere 20 h gerührt. Dann wurden 250 ml Dichlormethan und portionsweise 25 g Natriumacetat zugegeben. Nach Filtration der rohen Lösung durch Celite® wurden 30 ml Wasser zugegeben und die Phasen getrennt. Die organische Phase wurde zur Trockne eingeengt. Das zurückbleibende Rohprodukt (27,88 g) wurde mit Ethylacetat/Methanol (4:1) an 300 g Kieselgel chromatographiert.
Ausbeute (GC): 29,2%.
Schmp.: 158-160 °C (aus *n*-Butylacetat).
- ¹H NMR (CDCl₃, 400 MHz) δ =: 1,35 (s, 9H);
3,43 (br. s, 2H);
3,74 (br. s, 2H);
5,32 (br. s, 1H);
5,53 (br. s, 1H);
7,06 (d, *J* = 6 Hz, 1H).
- ¹³C NMR (CDCl₃, 100 MHz) δ =: 28,9;
42,3;
42,8;
51,2;
105,9;
116,3 (¹*J*_{CF} *=* 288 Hz);
133,2;
154,5 (²*J*_{CF}=35 Hz);
163,7.
- MS: *m/z* =: 57 (100%), 279 (M⁺, 6,2%).

### Beispiel 43

### 4-Formyl-1-trifluoracetyl-1,4,5,6-tetrahydropyrazin-2-carbonsäure-tert-butylamid

Zu einer Lösung von 96,94 g (347,1 mmol) 1-Trifluoracetyl-1,4,5,6-tetrahydropyrazin-2-carbonsäure-*tert*-butylamid in 465,9 g (10,1 mol) Ameisensäure wurden innerhalb von 40 min bei 20 °C 49,60 g (416,9 mmol) Thionylchlorid zugegeben. Nach 2 h Reaktionsdauer bei 20 °C wurde das Gemisch bei 60 °C und 80 mbar am Rotationsverdampfer eingeengt und der braunschwarze Rückstand mit 400 ml Ethylacetat und 400 ml Wasser aufgenommen. Die wässrige Phase wurde noch zweimal mit je 250 ml Ethylacetat gewaschen und die vereinigten organischen Extrakte über 50,1 g Natriumsulfat getrocknet. Nach Filtration wurde die Lösung am Rotationsverdampfer eingeengt und der dickflüssige Rückstand mit 250 ml Diisopropylether gerührt. Der ausgefallene Feststoff wurde abfiltriert, zweimal mit je 100 ml Diisopropylether gewaschen und getrocknet.
Ausbeute: 96,76 g (91%) farblose Kristalle
Schmp.: 158,4-160,3 °C

NMR-Daten: Im ¹H- und im ¹³C NMR ist ein jeweils ein doppelter Signalsatz sichtbar. Die Signale des Hauptkonformeren sind mit * markiert, soweit eine Zuordnung möglich war.
- ¹H NMR (CDCl₃, 400 MHz) δ =: 8,40 (s, 1H)*;
8,14 (s, 1H);
7,41 (s, 1H);
7,14 (s,1H)*;
5,65 (br. s, 1H);
5,55 (br. s, 1H)*;
3,93-3,80 (m, 2×4H);
1,40 (s, 9H);
1,39 (s, 9H)*.
- ¹³C NMR (CDCl₃, 100 MHz) δ =: 161,69;
161,14;
160,49;
159,61;
122,76;
119,76;
117,42;
115,84 (¹*J*_{CF} = 288 Hz);
52,04;
52,00;
44,77;
41,83;
41,11;
28,62.

### Beispiel 44

### (S)-4-Formyl-1-(trifluoracetyl)piperazin-2-carbonsäure-tert-butylamid

In einem 160 ml Autoklaven wurden 20,6 g (67,0 mmol) 4-Formyl-1-trifluoracetyl-1,4,5,6-tetrahydropyrazin-2-carbonsäure-*tert*-butylamid, 26,2 mg (64,5 µmol) Bis(1,5-cyclooctadien)-rhodium(I)tetrafluoroborat und 41,8 mg (77 µmol) 1-[1(*R*)-(Di-*tert*-butylphosphino)ethyl]-2(*S*)-(diphenylphosphino)ferrocen unter Argon vorgelegt (S/C = 1000). Man gab 70 ml Äceton (entgast) zu und hydrierte 11 h bei 100°C unter 13-10 bar Wasserstoffdruck. Nach dem Abdestillieren des Lösungsmittel wurden 20,5 g (99%) der Titelverbindung als kristalliner Feststoff erhalten.
ee = 96,4%
Schmp.: 172,0-173,0°C
- ¹H NMR (DMSO-d₆, 400 MHz, 70 °C) δ =: 8,06 und 8,02 (2 s, 1H);
7,63 (br. "d", 1H);
4,90-2,95 (m, 7H);
1,28 und 1,23 (2 s, 9H).
- ¹³C NMR (DMSO-d₆, 100 MHz, 70 °C): Ausgewählte Signale:
δ = 166,94;
166,70;
161,05;
160,94;
156,22 (q, ²*J*_{CF} = 35 Hz, COCF₃);
116,32 (q, ^{I}*J*_{CF} = 288 Hz, CF₃).

### Beispiel 45

### (S)-1-(Trifluoracetyl)piperazin-2-carbonsäure-tert-butylamid-Schwefelsäuresalz(I:1)

In einem 50 ml Autoklaven wurden 2,50 g (8,95 mmol) 1-Trifluoracetyl-1,4,5,6-tetrahydropyrazin-2-carbonsäure-*tert*-butylamid, 7,5 mg (18 µmol) Bis(1,5-cyclooctadien)rhodium(I)-tetrafluoroborat und 11,5 mg (21 µmol) 1-[1(*R*)-(Di-*tert*-butylphosphino)ethyl)-2(*S*)-(diphenylphosphino)ferrocen unter Argon vorgelegt (Edukt : Katalysator = 486). Dann gab man 0,89 g (9,08 mmol) Schwefelsäure gelöst in 20 ml entgastem Tetrahydrofuran zu. Anschliessend wurde 18 h bei 20°C unter 13-10 bar Wasserstoffdruck hydriert. Nach dem Abdestillieren des Lösungsmittels erhielt man 3,42 g (>99 %) der Titelverbindung als weisses Pulver.
ee = 79,5%
- ¹H NMR(DMSO-d₆, 400 MHz) δ =: 9,60-8,30 (m, 2H);
7,88 (br. s, 1H);
4,95-3,05 (m, 7H);
1,32 (s, 9H).
- ¹³C NMR (DMSO-d₆, 100 MHz): Ausgewählte Signale:
δ = 166,94 (CONH);
115,90 (q, 1*J*_{CF} = 288 Hz, CF₃).

### Beispiel 46

### (S)-1-(Trifluoracetyl)piperazin-2-carbonsäure-tert-butylamid

In einem 50 ml Autoklaven wurden 2,50 g (8,95 mmol) 1-Trifluoracetyl-1,4,5,6-tetrahydropyrazin-2-carbonsäure-*tert*-butylamid, 7,5 mg (18 µmol) Bis(1,5-cyclooctadien)rhodium(I)-tetrafluoroborat und 11,5 mg (21 µmol) 1-[1(*R*)-(Di-*tert*-butylphosphino)ethyl]-2(*S*)-(diphenylphosphino)ferrocen unter Argon vorgelegt (Edukt : Katalysator = 486). Man gab 20 ml entgastes Ethylacetat hinzu und hydrierte 18 h bei 90°C unter 13-10 bar Wasserstoffdruck. Nach dem Abdestillieren des Lösungsmittels erhielt man 2,50 g der rohen Titelverbindung als hellgelbes Pulver.
ee = 47,2%
- ¹H NMR (CDCl₃, 400 MHz): (Nur die Signale des Hauptisomeren sind angegeben.)
- δ =: 5,88 (br. s, 1H);
4,68-4,65 ("d", 1H);
3,85-3,78 ("d", 1H);
3,54-3,49 ("d", 1H);
3,41-3,32 ("dt", 1H);
3,11-3,06 ("d", 1H);
2,90-2,78 (m, 2H);
2,07 (s, 1H);
1,34 (s, 9H).

### Beispiel 47

### (S)-1-(Trifluoracetyl)piperazin-2-carbonsäure-tert-butylamid-BF₃-Addukt

In einem 50 ml Autoklaven wurden 2,50 g (8,95 mmol) 1-Trifluoracetyl-1,4,5,6-tetrahydropyrazin-2-carbonsäure-*tert*-butylamid, 7,5 mg (18 µmol) Bis(1,5-cyclooctadien)rhodium(I)-tetrafluoroborat und 11,5 mg (21 µmol) 1-[1(*R*)-(Di-*tert*-butylphosphino)ethyl]-2(*S*)-(diphenylphosphino)ferrocen unter Argon vorgelegt (Edukt:Katalysator = 488). Man gab eine Lösung von 0,71 g (6,83 mmol) Bortrifluorid-dihydrat in 20 ml entgastem Tetrahydrofuran zu. Anschliessend wurde 18 h bei 90°C unter 13-10 bar Wasserstoffdruck hydriert. Nach dem Abdestillieren des Lösungsmittels erhielt man 3,09 g der rohen Titelverbindung als weisses Pulver.
ee=51,6%

### Beispiel 48

### (S)-4-Formylpiperazin-2-carbonsäure-tert-butylamid

Zu 15,0 g (48,5 mmol) (*S*)-4-Formyl-1-(trifluoracetyl)piperazin-2-carbonsäure-*tert*-butylamid in 130 ml Isobutylalkohol wurde eine Lösung von 25,5 g (184,5 mmol) Kaliumcarbonat in 130 ml Wasser gegeben und das Gemisch 1 h auf 60-65 °C erhitzt Nach der Phasentrennung wurde die wässrige Phase mit 100 ml Isobutylalkohol extrahiert und die vereinigten organischen Phasen eingedampft. Es wurden 14,10 g der rohen (noch lösungsmittelhaitigen) Titelverbindung als hellgelbes Öl erhalten.
MS: *m/z* = 213 (M⁺, 1%), 198 (1%), 155 (2%), 113 (100%), 85 (36%), 56 (89%).
- ¹H NMR (CDCl₃, 400 MHz) δ =: 8,06 und 8,04 (2 br. s, 1H);
6,86 und 6,47 (2 br. s, 1H);
4,32-4,27 und 3,76-3,61 (3 m, 2H);
3,48-3,41 ("dd", 1H);
3,28-3,15 (m, 2H);
3,07-2,90 (m, 1H);
2,80-2,71 (m, 1H);
1,35 (s, 9H).

### Beispiel 49

### (S)-4-Formylpiperazin-2-carbonsäure-tert-butylamid-Essigsäuresalz (1:1)

Zu 21,58 g (101,2 mmol) (*S*)-4-Formylpiperazin-2-carbonsäure-*tert*-butylamid in 120 ml

Tetrahydrofuran wurden 120 ml Essigsäure gegeben und das Gemisch zur Trockne eingedampft. Anschliessend wurden 100 ml Toluol zugegeben und das Gemisch am Rotationsverdampfer eingeengt. Nach der Zugabe von 100 ml 1,4-Dioxan wurde nochmals eingeengt, wobei ein Feststoff ausfiel. Der Rückstand wurde mit 150 ml Ethylacetat aufgeschlämmt und abfiltriert. Nach Waschen mit Ethylacetat und Diethylether und Trocknen wurden 20,10 g (73%) der Titelverbindung als weisser, kristalliner Feststoff erhalten.
- ¹H NMR (DMSO-d₆, 400 MHz) δ =: 8,00 und 7,98 (2 s, 1H);
7,37 und 7,33 (2 br. s, 1H);
4,09-4,03 und 3,68-3,43 (4 "d", 2H);
3,21-3,11 (m, 1H);
3,08-2,99 (m, 1H);
2,96-2,87 (m, 1H);
2,83-2,76 und 2,70-2,61 (2 m, 1 H);
2,59-2,46 (m, 1H);
1,91 (s, 3H);
1,26 (s, 9H).

### Beispiel 50

### (S)-1-(Trifluoracetyl)piperazin-2-carbonsäure-tert-butylamid

Zu 32,0 g (103 mmol) (*S*)-4-Formyl-1-(trifluoracetyl)piperazin-2-carbonsäure-tert-butylamid in 200 ml Aceton wurde eine Mischung von 50,5 g (430 mmol) 32%iger Salzsäure und 50 ml Wasser gegeben und 2,5 h auf 60-65 °C erhitzt. Nach dem Abkühlen auf 20 °C wurde mit Triethylamin so neutralisiert, dass die Temperatur unter 30 °C blieb. Das Lösungsmittel wurde abgedampft und 100 ml Wasser und 200 ml Methylisobutylketon zugegeben. Bei 50 °C wurden die Phasen getrennt und die wässrige Phase noch zweimal mit je 100 ml Methylisobutylketon extrahiert. Die vereinigten organischen Extrakte wurden über Magnesiumsulfat gerührt und zur Trockne eingedampft. Es wurden 23,5 g (80%) der Titelverbindung als leicht rötlicher Feststoff erhalten. Umkristallisation aus Toluol/Diisopropylether ergab die Titelverbindung als weisses, kristallines Pulver.
Schmp.: 143,5-144,2 °C
¹H NMR (CDCl₃, 400 MHz) (Nur die Signale des Hauptisomeren sind angegeben)
- δ=: 5,88 (br. s, 1H);
4,68-4,65 ("d", 1H);
3,85-3,78 ("d", 1H);
3,54-3,49 ("d", 1H);
3,41-3,32 ("dt", 1H);
3,11-3,06 ("d", 1H);
2,90-2,78 (m, 2H);
2,07 (s, 1H);
1,34 (s, 9H).

### Beispiel 51

### (S)-3-(tert-Butylcarbamoyl)piperazin-1-carbonsäure-tert-butylester [=(S)-4-(tert-Butoxycarbonyl)piperazin-2-carbonsäure-tert-butylamid]

In einem 11 Doppelmantelrührwerk wurde das Reaktionsgemisch aus der asymmetrischen Hydrierung von 50,0 g (162,7 mmol) 4-Formyl-1-trifluoracetyl-1,4,5,6-tetrahydropyrazin-2-carbonsäure-*tert*-butylamid vorgelegt, mit einer Mischung von 64,75 g (567 mmol) 32%iger Salzsäure und 130 g Wasser versetzt und 2 h zum Rückfluss (*ca*. 60 °C) erhitzt. Das Reaktionsgemisch wurde auf 20 °C abgekühlt und so mit 73,76 g (729 mmol) Triethylamin versetzt, dass die Temperatur nicht über 30 °C stieg. Anschliessend gab man innerhalb 1 h bei 30 °C eine Lösung von 31,82 g (145,8 mmol) Di-*tert*-butyl-dicarbonat in 35 g Aceton zu. Nach 15 min Nachreaktionszeit destillierte man das Aceton bei max. 40 °C und 100 mbar ab. Zum Rückstand wurden 300 ml Methanol und eine Lösung von 45,36 g (1,13 mol) Natriumhydroxid in 65 g Wasser gegeben, das Gemisch 2 h zum Rückfluss erhitzt und anschliessend das überschüssige Methanol bei maximal 48 °C und 100 mbar abdestilliert. Danach gab man 100 ml Wasser und 200 ml Methylcyclohexan zu, erhitzte 5 min auf 55 °C und trennte die Phasen. Die wässrige Phase wurde noch zweimal mit je 75ml Methylcyclohexan von 55°C gewaschen und die vereinigten organischen Phasen bei 50-60 °C teilweise eingedampft, wobei die Kristallisation begann. Dann wurde auf 15 °C abgekühlt und die Temperatur 1 h gehalten. Nach einer weiteren Stunde bei 0 °C wurde filtriert und der Rückstand mit 100 ml kaltem Diisopropylether gewaschen. Nach dem Trocknen wurden 35,45 g (77%) der Titelverbindung als weisser, kristalliner Feststoff erhalten.
ee=98,7%
- ¹H NMR (400 MHz, CDCl₃) δ =: 6,75-6,45 (br. s, 1H);
4,13-4,02 (m, 1H);
3,90-3,70 (br. s, 1H);
3,20 (dd, *J*= 3,7, 9,3 Hz, 1H);
3,05-2,80 (m, 3H);
2,80-2,70 (m, 1H);
2,05-1,90 (br. s, 1H);
1,47 (s, 9H);
1,35 (s, 9H).
- ¹³C NMR (100 MHz, CDCl₃) δ =: 170,27;
154,76;
80,11;
58,93;
50,93;
43,40;
44,17;
44,00;
28,75;
28,43.

## Patentansprüche

1. Verfahren zur Herstellung von optisch aktiven 2-Piperazincarbonsäurederivaten der allgemeinen Formel 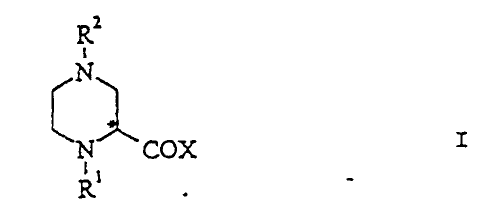 worin R¹ und R² unabhängig voneinander jeweils Wasserstoff, C₁₋₄-Alkyl, gegebenenfalls substituiertes C₁₋₆-Alkanoyl, C₂₋₆-Perfluoralkanoyl, Aroyl, Arylalkyl, C₁₋₆-Alkoxycarbonyl, Aryloxycarbonyl, Carbamoyl oder eine Aminoschutzgruppe bedeuten und X eine Hydroxygruppe, eine C₁₋₆-Alkoxygruppe oder eine Gruppe der Formel -NR³R⁴ ist, worin wiederum
(i) R³ und R⁴ unabhängig voneinander Wasserstoff, C₁₋₆-Alkyl, C₃₋₆-Cycloalkyl, Aryl oder eine Aminoschutzgruppe bedeuten oder
(ii) R³ und R⁴ zusammen mit dem Stickstoffatom einen gegebenenfalls substituierten 5- oder 6-gliedrigen gesättigten heterocyclischen Ring bilden oder
(iii) R³ Wasserstoff und R⁴ eine Gruppe der Formel 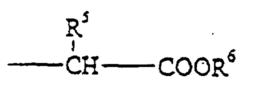 ist, worin R⁵ Wasserstoff oder die Seitengruppe einer Aminosäure und R⁶ Wasserstoff, C₁₋₄-Alkyl oder Aryl ist, **dadurch gekennzeichnet, dass** ein entsprechendes 1,4,5,6-Tetrahydro-2-pyrazincarbonsäurederivat der allgemeinen Formel 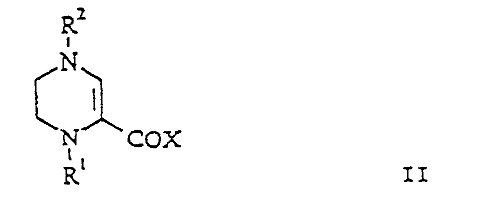
worin R¹, R² und X die oben genannten Bedeutungen haben, in Gegenwart eines katalytisch wirksamen, aus einem Rh(I)-Komplex und einem optisch aktiven Metallocenylphosphin gebildeten optisch aktiven Rhodiumkomplexes asymmetrisch hydriert wird

2. Verfahren nach Anspruch 1, **dadurch gekennzeichnet, dass** als 1,4,5,6-Tetrahydro-2-pyrazincarbonsäurederivat II eine Verbindung eingesetzt wird, in der R¹ und/oder R² jeweils C₁₋₆-Alkanoyl, C₂₋₆-Perfluoralkanoyl, *tert*-Butoxycarbonyl oder Benzyloxycarbonyl ist.

3. Verfahren nach Anspruch 1 oder 2, **dadurch gekennzeichnet, dass** als 1,4,5,6-Tetrahydro-2-pyrazincarbonsäurederivat II ein Amid eingesetzt wird, in dem X eine Gruppe der Formel -NR³R⁴ ist und R³ und R⁴ die in Anspruch 1 genannten Bedeutungen haben,

4. Verfahren nach Anspruch 3, **dadurch gekennzeichnet, dass** R³ Wasserstoff und R⁴ eine C₁₋₆-Alkylgruppe ist.

5. Verfahren nach Anspruch 4, **dadurch gekennzeichnet, dass** R⁴ eine *tert*-Butylgruppe ist.

6. Verfahren nach einem der Ansprüche 1 bis 5, **dadurch gekennzeichnet, dass** als optisch aktives Metallocenylphosphin eine Verbindung der allgemeinen Formel 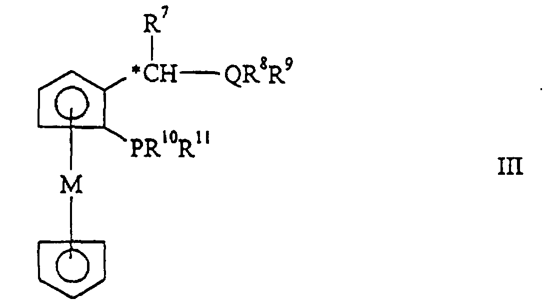 worin M Eisen(II) oder Ruthenium(II), Q Stickstoff oder Phosphor, R⁷ eine C₁₋₄-Alkylgruppe und R⁸ bis R¹¹ unabhängig voneinander C₁₋₈-Alkyl, C₅₋₈-Cycloalkyl, Phenyl oder substituiertes Phenyl bedeuten, eingesetzt wird.

7. Verfahren nach Anspruch 6, **dadurch gekennzeichnet, dass** M Eisen(II) ist.

8. Verfahren nach Anspruch 6 oder 7, **dadurch gekennzeichnet, dass** Q Phosphor ist.

9. Verfahren nach einem der Ansprüche 6 bis 8, **dadurch gekennzeichnet, dass** R⁷ Methyl ist und R⁸ und R⁹ gleich sind und *tert*-Butyl oder Cyclohexyl bedeuten.

10. Verfahren nach einem oder mehreren der Ansprüche 5 bis 9, **dadurch gekennzeichnet, dass** als Rh(I)-Komplex ein neutraler Komplex der allgemeinen Formel
[Rh(L)A]₂ IV
worin L ein C₄₋₁₂-Dien oder zwei C₂₋₁₂-Alkenmoleküle bedeutet und A Chlor, Brom oder lod ist, eingesetzt wird.

11. Verfahren nach einem oder mehreren der Ansprüche 5 bis 9, **dadurch gekennzeichnet, dass** als Rh(I)-Komplex ein kationischer Komplex der allgemeinen Formel
[Rh(L)₂]⁺B⁻ V
worin L ein C₄₋₁₂-Dien oder zwei C₂₋₁₂-Alkenmoleküle bedeutet und B⁻ das Anion einer Sauerstoffsäure oder einer Komplexsäure ist, eingesetzt wird.

12. Verfahren nach Anspruch 10 oder 11, **dadurch gekennzeichnet, dass** L Norbomadien oder 1,5-Cyclooctadien bedeutet.

13. 1,4,5,6-Tetrahydro-2-pyrazincarbonsäurederivate der allgemeinen Formel 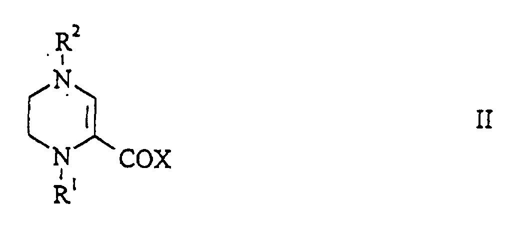 worin R¹, R² und X die in Anspruch 1 genannten Bedeutungen haben, mit der Massgabe, dass nicht R¹ und R² gleichzeitig Wasserstoff sind.

14. 1,4,5,6-Tetrahydro-2-pyrazincarbonsäurederivate nach Anspruch 13, **dadurch gekennzeichnet, dass** R¹ und/oder R² gegebenenfalls substituiertes C₁₋₆-Alkanoyl, C₂₋₆-Perfluoralkanoyl, *tert*-Butoxycarbonyl oder Benzyloxycarbonyl ist.

15. 1,4,5,6-Tetrahydro-2-pyrazincarbonsäurederivate nach Anspruch 14, **dadurch gekennzeichnet, dass** R¹ und/oder R² Formyl, Acetyl oder Trifluoracetyl ist.

16. Verfahren zur Herstellung von 1,4,5,6-Tetrahydro-2-pyrazincarbonsäurederivaten der allgemeinen Formel 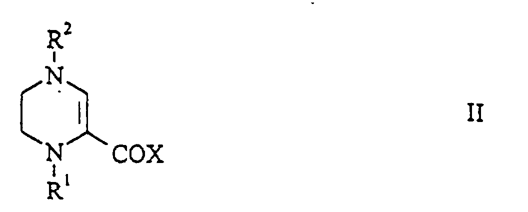 worin R¹, R² und X die in Anspruch 1 genannten Bedeutungen haben, mit der Massgabe, dass nicht R¹ und R² gleichzeitig Wasserstoff sind,
**dadurch gekennzeichnet, dass** ein Pyrazincarbonsaurederivat der allgemeinen Formel 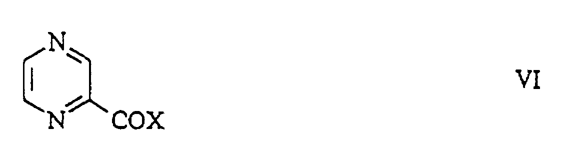 worin X die genannte Bedeutung hat, mit Wasserstoff an einem Palladiumkatalysator zum 1,4,5,6-Tetrahydro-2-pyrazincarbonsäurederivat der allgemeinen Formel 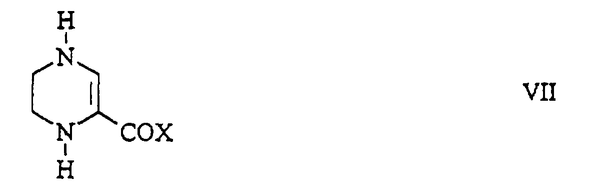 hydriert und anschliessend nach einer bekannten Methode in die an N¹ und/oder N⁴ substituierte Verbindung der allgemeinen Formel II überführt wird.

## Claims

1. A process for the preparation of optically active 2-piperazinecarboxylic acid derivatives of the general formula 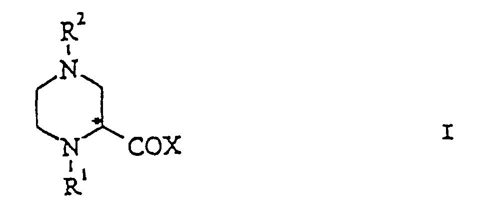 wherein R¹ and R² each independently represent hydrogen, C₁₋₄ alkyl, optionally substituted C₁₋₆ alkanoyl, C₂₋₆ perfluoroalkanoyl, aroyl, arylalkyl, C₁₋₆ alkoxycarbonyl, aryloxycarbonyl, carbamoyl or an amino protective group, and X is a hydroxy group, a C₁₋₆ alkoxy group or a group of the formula -NR³R⁴, wherein in turn
(i) R³ and R⁴ independently represent hydrogen, C₁₋₆ alkyl, C₃₋₆ cycloalkyl, aryl or an amino protective group, or
(ii) R³ and R⁴ together with the nitrogen atom form an optionally substituted 5- or 6-membered saturated heterocyclic ring, or
(iii) R³ is hydrogen and R⁴ is a group of the formula 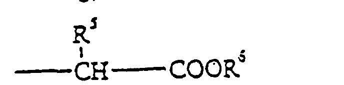
wherein R⁵ is hydrogen or the side group of an amino acid and R⁶ is hydrogen, C₁₋₄ alkyl or aryl, **characterized in that** a corresponding 1,4,5,6-tetrahydro-2-pyrazinecarboxylic acid derivative of the general formula 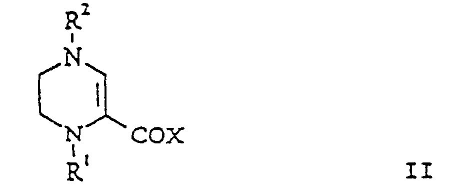 wherein R¹, R² and X have the above indicated meanings, is asymmetrically hydrogenated in the presence of a catalytically effective, optically active rhodium complex formed of a Rh(I) complex and an optically active metallocenyl phosphine.

2. Process according to claim 1, **characterized in that** as the 1,4,5,6-tetrahydro-2-pyrazinecarboxylic acid derivative II a compound is used in which R¹ and/or R² each are C₁₋₆ alkanoyl, C₂₋₆ perfluoroalkanoyl, *tert*-butoxycarbonyl or benzyloxycarbonyl.

3. Process according to claim 1 or 2, **characterized in that** as the 1,4,5,6-tetrahydro-2-pyrazinecarboxylic acid derivative II an amide is used in which X is a group of the formula -NR³R⁴ and R³ and R⁴ have the meanings indicated in claim 1.

4. Process according to claim 3, **characterized in that** R³ is hydrogen and R⁴ is a C₁₋₆ alkyl group.

5. Process according to claim 4, **characterized in that** R⁴ is a *tert*-butyl group.

6. Process according to any one of claims 1 to 5, **characterized in that** as the optically active metallocenyl phosphine a compound of the general formula 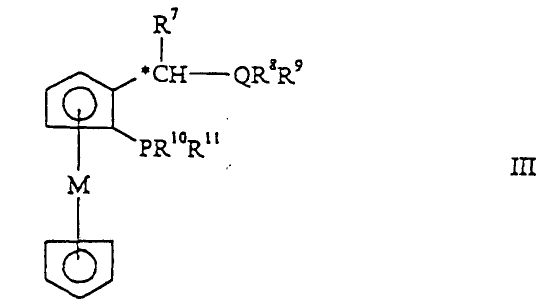 is used, wherein M represents iron(II) or ruthenium(II), Q represents nitrogen or phosphorus, R⁷ represents a C₁₋₄ alkyl group, and R⁸ to R¹¹ independently represent C₁₋₈ alkyl, C₅₋₈ cycloalkyl, phenyl or substituted phenyl.

7. Process according to claim 6, **characterized in that** M is iron(II).

8. Process according to claim 6 or 7, **characterized in that** Q is phosphorus.

9. Process according to any one of claims 6 to 8, **characterized in that** R⁷ is methyl, and R⁸ and R⁹ are identical and represent *tert*-butyl or cyclohexyl.

10. Process according to one or more of claims 5 to 9, **characterized in that** as a Rh(l) complex a neutral complex of the general formula
[Rh(L)A]₂ IV
is used, wherein L represents a C₄₋₁₂ diene or two C₂₋₁₂ alkene molecules and A is chlorine, bromine or iodine.

11. Process according to one or more of claims 5 to 9, **characterized in that** as a Rh(I) complex a cationic complex of the general formula
[Rh(L)₂]⁺B⁻ V
is used, wherein L represents a C₄₋₁₂ diene or two C₂₋₁₂-alkene molecules, and B⁻ is the anion of an oxygen acid or of a complex acid.

12. Process according to claim 10 or 11, **characterized in that** L represents norbornadiene or 1,5-cyclooctadiene.

13. 1,4,5,6-Tetrahydro-2-pyrazinecarboxylic acid derivatives of the general formula 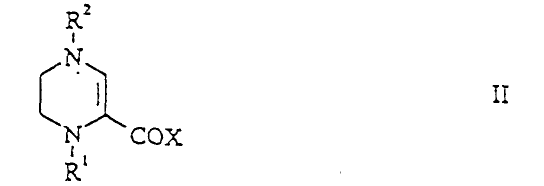 wherein R¹, R² and X have the meanings indicated in claim 1, under the proviso that R¹ and R² are not simultaneously hydrogen.

14. 1,4,5,6-Tetrahydro-2-pyrazinecarboxylic acid derivatives according to claim 13, **characterized in that** R¹ and/or R² is optionally substituted C₁₋₆ alkanoyl, C₂₋₆ perfluoroalkanoyl, *tert*-butoxycarbonyl or benzyloxycarbonyl.

15. 1,4,5,6-Tetrahydro-2-pyrazinecarboxylic acid derivatives according to claim 14, **characterized in that** R¹ and/or R² is formyl, acetyl or trifluoroacetyl.

16. Process for the preparation of 1,4,5,6-tetrahydro-2-pyrazinecarboxylic acid derivatives of the general formula 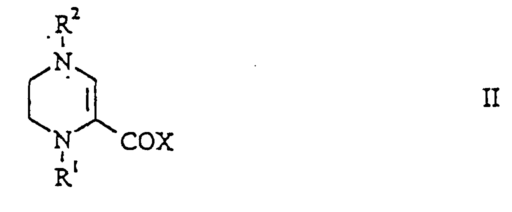 wherein R¹, R² and X have the meanings indicated in claim 1, under the proviso that R¹ and R² are not simultaneously hydrogen, **characterized in that** a pyrazinecarboxylic acid derivative of the general formula 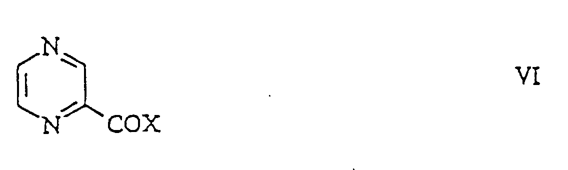 wherein X has the indicated meaning, is hydrogenated with hydrogen on a palladium catalyst to give the 1,4,5,6-tetrahydro-2-pyrazinecarboxylic acid derivative of the general formula 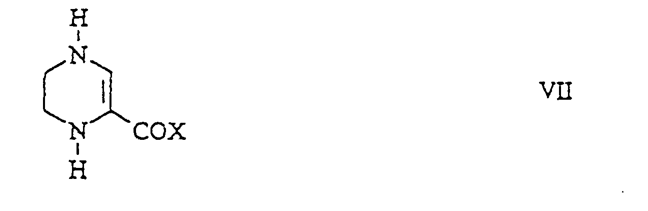 and subsequently is converted in accordance with a known method into the compound of the general formula II substituted at N¹ and/or N⁴.

## Revendications

1. Procédé pour la préparation des dérivés de l'acide 2-pipérazinique optiquement actif de formule générale 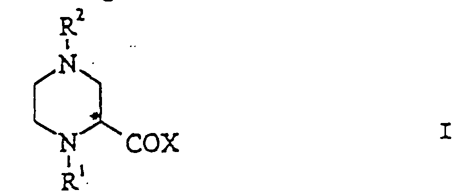 dans laquelle R¹ et R², indépendants l'un de l'autre, représentent chacun un atome d'hydrogène, un alkyle de C₁ à C₄, un alkanoyle de C₁ à C₆ éventuellement substitué, un perfluoralkanoyle de C₂ à C₆, un aroyle, un arylalkyle, un alkoxycarbonyle de C₁ à C₆, un aryloxycarbonyle, un carbamoyle ou un groupe amino-protecteur et X représente un groupe hydroxy, un groupe alkoxy de C₁ à C₆ où un groupe de formule -NR³R⁴, dans laquelle d'autre part
(i) R³ et R⁴, indépendants l'un de l'autre, représentent un atome d'hydrogène, un alkyle de C₁ à C₆, un cycloalkyle de C₃ à C₆, un aryle ou un groupe amino-protecteur, ou
(ii) R³ et R⁴, en commun avec l'atome d'azote, constituent un anneau hétérocyclique saturé éventuellement substitué par 5 ou 6 membres, ou
(iii) R³ est un atome d'hydrogène et R⁴ un groupe de formule 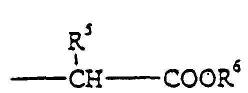
dans laquelle R' est un atome d'hydrogène ou le groupe latéral d'un aminoacide et R⁶ un atome d'hydrogène , un alkyle de C₁ à C₄, ou un aryle, **caractérisé en ce qu'**on soumet un dérivé de l'acide 1,4,5,6-tétrahydro-2-pyrazinique de formule générale 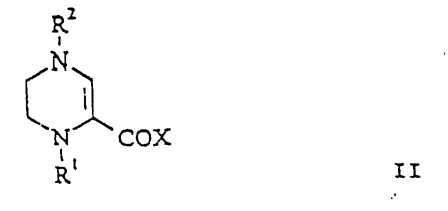 dans laquelle R¹, R² et X ont les significations indiquées ci-dessus, à une hydrogénation asymétrique en présence d'un complexe de rhodium optiquement actif, efficace de manière catalytique et constitué d'un complexe Rh(I) et d'un phosphine métallocénique optiquement actif.

2. Procédé selon la revendication 1, **caractérisé en ce que** l'on utilise comme dérivé II de l'acide 1,4,5,6-tétrahydro-2-pyrazinique un composé dans lequel R¹ et/ou R² soit chacun un alkanoyle de C₁ à C₆, un perfluoralkanoyle de C₂ à C₆, un tert-butoxycarbonyle ou un benzyloxycarbonyle.

3. Procédé selon la revendication 1 ou 2, **caractérisé en ce que** l'on utilise comme dérivé II de l'acide 1,4,5,6-tétrahydro-2-pyrazinique un amide dans lequel X représente un groupe de la formule -NR³R⁴, où R³ et R⁴ ont les significations indiquées dans la revendication 1.

4. Procédé selon la revendication 3, **caractérisé en ce que** R³ est un atome d'hydrogène et R⁴ un groupe alkyle de C₁ à C₆.

5. Procédé selon la revendication 4, **caractérisé en ce que** R⁴ est un groupe tert-butyle.

6. Procédé selon l'une des revendications 1 à 5, **caractérisé en ce que** l'on utilise comme phosphine métallocénique optiquement actif un composé de formule générale 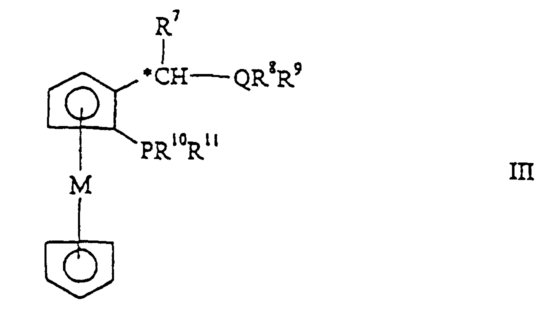 dans laquelle M représente le fer(II) ou le ruthenium(II), Q représente l'azote ou le phosphore, R⁷ représente un groupe alkyle de C₁ à C₄ et R⁸ jusqu'à R¹¹, indépendants les uns des autres, représentent un alkyle de C₁ à C₈, un cycloalkyle de C₅ à C₈, un phényle ou un phényle substitué.

7. Procédé selon la revendication 6, **caractérisé en ce que** M représente le fer(II).

8. Procédé selon la revendication 6 ou 7, **caractérisé en ce que** Q représente le phosphore.

9. Procédé selon l'une des revendications 6 à 8, **caractérisé en ce que** R⁷ représente un méthyle et R⁸ et R⁹ sont identiques et représentent le tert-butyle ou le cyclohexyle.

10. Procédé selon une ou plusieurs des revendications 5 à 9, **caractérisé en ce que** l'on utilise comme complexe Rh(I) un complexe neutre de formule générale
[Rh(L)A]₂
dans laquelle L représente un diène de C₄ à C₁₂ ou deux molécules d'alkène de C₂ à C₁₂ et A représente le chlore, le brome ou l'iode.

11. Procédé selon une ou plusieurs des revendications 5 à 9, **caractérisé en ce que** l'on utilise comme complexe Rh(I) un complexe cationique de formule générale
[Rh(L)₂]⁺B⁻
dans laquelle L représente un diène de C₄ à C₁₂ ou deux molécules d'alkène de C₂ à C₁₂ et B⁻ représente l'anion d'un acide d'oxygène ou d'un acide complexe.

12. Procédé selon la revendication 10 ou 11, **caractérisé en ce que** L représente un norbornadiène ou un 1,5-cyclooctadiéne.

13. Dérivés de l'acide 1,4,5,6-tétrahydro-2-pyrazinique de formule générale 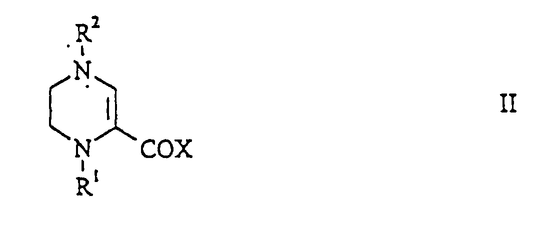 dans laquelle R¹, R² et X ont les significations indiquées dans la revendication 1, sous réserve que R¹ et R² ne représentent pas en même temps un atome d'hydrogène.

14. Dérivés de l'acide 1,4,5,6-tétrahydro-2-pyrazinique selon la revendication 13, **caractérisé en ce que** R¹ et/ou R² représentent un alkanoyle de C₁ à C₆ éventuellement substitué, un perfluoralkanoyle de C₂ à C₆, un tert-butoxycarbonyle ou un benzyloxicarbonyle.

15. Dérivés de l'acide 1,4,5,6-tétrahydro-2-pyrazinique selon la revendication 14, **caractérisé en ce que** R' et/ou R² représente un formyle, un acétyle ou un trifluoracétyle.

16. Procédé pour la préparation des dérivés de l'acide 1,4,5,6-tétrahydro-2-pyrazinique de formule générale 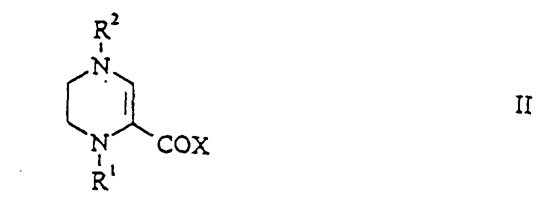 dans laquelle R¹, R² et X ont les significations indiquées dans la revendication 1, sous réserve que R¹ et R² ne représentent pas en même temps un atome d'hydrogène, **caractérisé en ce qu'**un dérivé de l'acide pyrazinique de formule générale 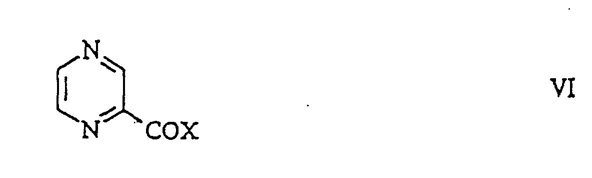 dans laquelle X a la signification indiquée, est soumis à une hydrogénation avec de l'hydrogène par un catalyseur de paladium pour obtenir un dérivé de l'acide 1,4,5,6-tétrahydro-2-pyrazinique de formule générale 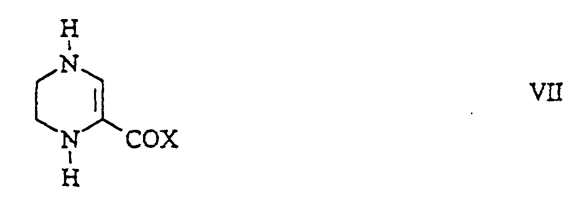 et est ensuite converti selon une méthode bien connue au composé de formule générale II substitué à N¹ et/ou N⁴.
